# EUROPÄISCHE PATENTSCHRIFT

(11) **EP 1 140 761 B1**
(45) Veröffentlichungstag und Bekanntmachung des Hinweises auf die Patenterteilung: **08.10.2003**
(21) Anmeldenummer: 00900032.4
(22) Anmeldetag: 11.01.2000
(51) Int. Cl.: C07C 49/84, C07C 45/46, C07C 45/63, C07C 45/71, C07C 45/64, C07C 323/22, C07D 333/62, C08F 2/50

(54) **BENZOPHENONE UND IHRE VERWENDUNG ALS PHOTOINITIATOREN**
BENZOPHENONES AND THE USE THEREOF AS PHOTOINITIATORS
BENZOPHENONES ET LEUR UTILISATION COMME PHOTO-AMORCEURS

(30) Priorität: 12.01.1999 CH 4799
(43) Veröffentlichungstag der Anmeldung: 10.10.2001
(73) Patentinhaber: Clariant Finance (BVI) Limited, Road Town, Tortola (VG)
(72) Erfinder: AVAR, Lajos, CH-4105 Biel-Benken (CH); BÄR, René, CH-4054 Basel (CH); SANAHUJA, Victor, CH-4106 Therwil (CH)
(74) Vertreter: D'haemer, Jan Constant
(86) Internationale Anmeldenummer: IB0000024
(87) Internationale Veröffentlichungsnummer: WO00041990

(56) Entgegenhaltungen:
- EP-A- 0 003 002
- FR-A- 2 391 183
- FR-A- 2 627 486
- GB-A- 2 025 991
- US-A- 4 582 862
- CHEMICAL ABSTRACTS, vol. 114, no. 16, 22. April 1991 (1991-04-22) Columbus, Ohio, US; abstract no. 144220, ARAGONES APODACA R ET AL: "Manufacture of 1-hydroxycyclohexyl aryl ketones for use as photoinitiators" XP002131559 & ES 2 011 728 A (MICROQUIMICA NAVARRA S. A.;SPAIN)

## Beschreibung

Die Erfindung hat neue Verbindungen zum Gegenstand, die als Photoinitiatoren bzw. Photosensibilisatoren verwendet werden können, ein Verfahren zur Herstellung dieser Verbindungen und deren Verwendung als Photoinitiatoren bzw. Photosensibilisatoren.

Aus dem Stand der Technik sind symmetrisch substituierte Ketonverbindungen als Photoinitatoren bekannt. EP 0 003 002 offenbart symmetrisch substituierte Ketonverbindungen, insbesondere Diisopropanolverbindungen (Nr. 21 und 23 auf Seite 38) als Photoinitiatoren für phatopolymerisierbare Systeme. GB 2 025 991 offenbart symmetrisch substituierte Diphenylketonverbindungen (Nr. 14 und 15 auf Seite 5) als Photoinitiatoren in photopolymerisierbaren Systemen.

Die neuen Verbindungen entsprechen der allgemeinen Formel (I) 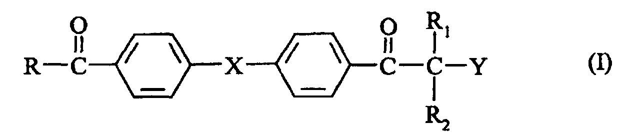 worin
- R: für Phenyl, mit C₁₋₄Alkyl, C₁₋₄Alkoxy oder Halogen substituiertes Phenyl, Naphthyl oder einen aromatischen Ring mit Heteroatomen steht;
- X: O, S bedeutet;
- R₁ und R₂: je für einen Methylrest oder R₁ und R₂ gemeinsam für einen C₄₋₈Alkylenrest stehen;
- Y: Hydroxy, C₁₋₁₂Alkoxy, C₁₋₄Akylamino, Di-C₁₋₄Alkylamino oder ein am Stickstoff gebundenen Piperidin- oder Morpholinring bedeutet.

Bevorzugt steht R für einen unsubstituierten Phenylring oder einen mit Chlor oder C₁₋₄Alkyl substituierten Phenylring, X für Sauerstoff, R₁ und R₂ je für einen Methylrest und Y für Hydroxy oder Morpholin.

Die Verbindungen der Formel (I) werden hergestellt,indem man Verbindungen der Formel(II) 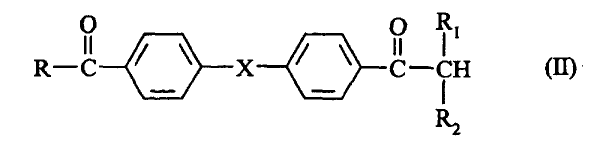 worin R, X, R₁ und R₂ die oben angegebenen Bedeutungen haben, halogeniert und den Halogenrest mit entsprechenden Verbindungen gegen die Gruppe Y austauscht.

Vorzugsweise verwendet man elementares Brom und tauscht den Bromrest im alkalischen Medium gegen Hydroxy, Alkoxy oder Amino aus.

Die Verbindungen der Formel (II) sind ebenfalls neu und können durch Umsetzung von Verbindungen der Formel (III) 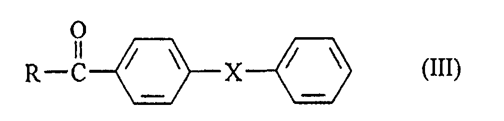 worin R und X die oben angegebenen Bedeutungen haben, mit Verbindungen der Formel (IV) 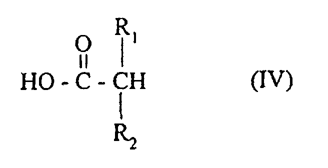 bzw. ihren reaktiven Derivaten, worin R₁ und R₂ die oben angegebenen Bedeutungen haben, in Anwesenheit von passenden Katalysatoren, z.B. AlCl₃, hergestellt werden.

Alle Verfahrensschritte sind an und für sich bekannte Umsetzungen und können analog zu beschriebenen Verfahren unter bekannten Reaktionsbedingungen durchgeführt werden.

Die neuen Verbindungen (1) sind Photoinitiatoren bzw. Photosensibilisatoren für photopolymerisierbare ungesättigte Verbindungen.

Solche Verbindungen sind beispielsweise ungesättigte Monomere wie Ester von Acryl- oder Methacrylsäure, z.B. Methyl-, Aethyl-, n- oder tert.Butyl-, Isooctyl- oder Hydroxyäthylacrylat, Methyl- oder Aethylmethacrylat, Aethylendiacrylat, Neopentyldiacrylat, Trimethylolpropantrisacrylat. Pentaerythrit-tetraacrylat oder Pentaerythrit-trisacrylat; Acrylnitril, Methacrylnitril, Acrylamid, Methacrylamid, N-substituierte (Meth)acrylamide; Vinylester wie z.B. Vinylacetat, -propionat, -acrylat oder -succinat; sonstige Vinylverbindungen wie Vinyläther, Styrol, Alkylstyrole, Halogenstyrole, Divinylbenzol, Vinylnaphthalin, N-Vinylpyrrolidon, Vinylchlorid oder Vinylidenchlorid; Allylverbindungen wie Diallylphthalat, Diallylmaleat, Triallylisocyanurat, Triallylphosphat oder Aethylenglycol-diallyläther und die Mischungen von solchen ungesättigten Monomeren.

Photopolymerisierbare Verbindungen sind weiterhin ungesättigte Oligomere oder Polymere und deren Mischungen mit ungesättigten Monomeren. Hierzu zählen thermoplastische Harze, die ungesättigte Gruppen wie Fumarsäureester, Allylgruppen oder Acrylat- oder Methacrylatgruppen enthalten. Meist sind diese ungesättigten Gruppen über funktionelle Gruppen an die Hauptkette dieser linearen Polymeren gebunden. Grosse Bedeutung haben Gemische von Oligomeren mit einfach und mehrfach ungesättigten Monomeren. Beispiele für solche Oligomere sind ungesättigte Polyester, ungesättigte Acrylharze und Isocyanatoder Epoxid- modifizierte Acrylatoligomere sowie Polyätheracrylatoligomere. Beispiele für mehrfach ungesättigte Verbindungen sind vor allem die Acrylate von Diolen und Polyolen, z.B. Hexamethylendiacrylat oder Pentaerythrit-tetracrylat. Auch als einfach ungesättigte Monomere werden Acrylate bevorzugt wie z.B. Butylacrylat, Phenylacrylat, Benzylacrylat, 2-Aethyl-hexylacrylat oder 2-Hydroxypropylacrylat. Man hat es durch Auswahl aus den verschiedenen Vertretern der drei Komponenten in der Hand, die Konsistenz des unpolymerisierten Gemisches sowie die Plastizität des polymerisierten Harzes zu variieren.

Neben diesen Dreikomponenten-Gemischen spielen bei den Polyesterharzen vor allem Zweikomponenten-Gemische eine grosse Rolle. Diese bestehen meist aus einem ungesättigten Polyester und einer Vinylverbindung. Die ungesättigten Polyester sind oligomere Veresterungsprodukte mindestens einer ungesättigten Dicarbonsäure wie z.B. Malein-, Fumar- oder Citraconsäure, und meist mindestens einer gesättigten Dicarbonsäure, wie z.B. Phthalsäure, Bernsteinsäure, Sebazinsäure oder Isophthalsäure, mit Glykolen wie z.B. Aethylenglykol, Propandiol-1,2, Di- oder Triäthylenglykol oder Tetramethylenglykol, wobei zur Modifizierung meist auch Monocarbonsäuren und Monoalkohole mitverwendet werden. Diese ungesättigten Polyester werden üblicherweise in einer Vinyl- oder Allylverbindung gelöst, bevorzugt wird hierfür Styrol verwendet.

Photopolymerisierbare Systeme, wie sie für die verschiedenen Zwecke verwendet werden, enthalten meist ausser den photopolymerisierbaren Verbindungen und dem Photoinitiator eine Reihe sonstiger Zusätze. So ist es vielfach üblich, thermische Inhibitoren zuzusetzen, die vor allem während der Herstellung der Systeme durch. Mischen der Komponenten vor einer vorzeitigen Polymerisation schützen sollen.

Zur Erhöhung der Dunkellagerstabilität können Kupferverbindungen wie Kupfernaphthenat, -stearat oder -octoat, Phosphorverbindungen wie. Triphenylphosphin, Tributylphosphin, Triäthylphosphit, Triphenylphosphit oder Tribenzylphosphat, quartäre Ammoniumverbindungen wie Tetramethylammoniumchlorid oder Trimethylbenzylammoniumchlorid oder Hydroxylaminderivate wie z.B. N-Diäthylhydroxylamin, zugesetzt werden.

Photopolymerisierbare Systeme enthalten weiterhin - je nach Verwendungszweck - Füllstoffe wie Kieselsäure, Talkum oder Gips, Pigmente, Farbstoffe, Fasern, Thixotropiemittel oder Verlaufhilfsmittel.

Femer können auch Kombinationen mit bekannten Photoinitiatoren, wie Benzoinäthern, Dialkoxyacetophenonen oder Benzilketalen, verwendet werden.

Besonders für die Photopolymerisation von dünnen Schichten und Druckfarben können Kombinationen des erfindungsgemässen Photoinitiators mit Aminen und/oder aromatischen Ketonen verwendet werden. Beispiele für Amine sind Triäthylamin, N-Methyldiäthanolamin, N-Dimethyläthanolamin oder p-Dimethylaminobenzoesäureester. Beispiele für Ketone sind Benzophenon, substituierte Benzophenonderivate, Michler's Ketone, Anthrachinon und Anthrachinonderivate, sowie Thioxanthon und dessen Derivate.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Gut geeignet ist der erfindungsgemässe Initiator auch für photohärtbare Systeme zur Herstellung von Druckplatten. Hierbei werden z.B. Gemische von löslichen linearen Polyamiden mit photopolymerisierbaren Monomeren, beispielsweise Acrylamide, und einem Photoinitiator verwendet. Filme oder Platten aus diesen Systemen werden über das Negativ (oder Positiv) der Druckvorlage belichtet und die ungehärteten Anteile anschliessend mit einem Lösungsmittel eruiert.

Ein weiteres Einsatzgebiet der UV-Härtung ist die Metallbeschichtung, beispielsweise bei der Lackierung von Blechen für Tuben, Dosen oder Flaschenverschlüssen, sowie die UV-Härtung von Kunststoffbeschichtungen, beispielsweise von Fussboden- oder Wandbelägen auf PVC-Basis.

Beispiele für die UV-Härtung von Papierbeschichtungen sind die farblose Lackierung von Etiketten, Schallplatten-Hüllen oder Buchumschlägen.

Der Photoinitiator wird für die angeführten Anwendungsgebiete zweckmässig in Mengen von 0,1 bis 20 Gew.-%, vorzugsweise etwa 0,5 bis 5 Gew.-%, bezogen auf das photopolymerisierbare bzw. vernetzbare System, angewendet. Unter System ist hierbei das Gemisch aus der photopolymerisierbaren bzw. vernetzbaren Verbindung, dem Photoinitiator und den sonstigen Füll- und Zusatzstoffen gemeint wie es in der jeweiligen Anwendung verwendet wird.

Der Zusatz des Photoinitiators zu den photopolymerisierbaren Systemen geschieht im allgemeinen durch einfaches Einrühren, da die meisten dieser Systeme flüssig oder gut löslich sind. Meist kommt es zu einer Lösung des Initiators, wodurch dessen gleichmässige Verteilung sowie die Transparenz der Polymerisate gewährleistet ist.

Die Polymerisation erfolgt nach den bekannten Methoden der Photopolymerisation durch Bestrahlung mit Licht, das reich an kurzwelliger Strahlung ist. Als Lichtquellen sind z.B. Quecksilbermitteldruck-, -hochdruck- und -niederdruckstrahler, sowie superaktinische Leuchtstoffröhren geeignet, deren Emissionsmaxima im Bereich zwischen 250 und 400 nm liegen.

### BEISPIELE

a) 13.7 g p-Phenoxy-benzophenon, 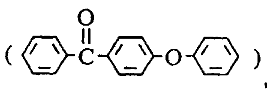 Smp. 69-71 °C werden in 100 ml Chlorbenzol zusammen mit 6 g Isobuttersäurechlorid vorgelegt und die Lösung bei 8 °C während 8 Stunden portionsweise mit 15 g AlCl₃ versetzt und über Nacht weitergerührt. Nach Zersetzung des AlCl₃ Komplexes mit Eiswasser, wurde die organische Phase abgetrennt, mit Wasser gewaschen und das Lösungsmittel abdestilliert. Der Rückstand wurde aus Isopropanol kristallisiert. Weisse Kristalle mit Smp. 97-99 °C

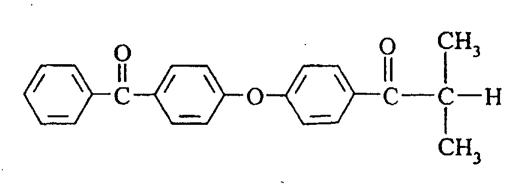

| | | | | |
|---|---|---|---|---|
| Elementaranalyse | Ber. | C: 80.2 % | H: 5.8% | 0:13.9% |
| | Gef. | C: 79.4 % | H: 5.8% | 0: 14.1% |

b) 8.6 g des Produktes aus der Stufe a) werden in 80 ml Eisessig vorgelegt und unter Rühren bei 15 °C während 1 Stunde mit 4.8 g Brom versetzt und die rotbraune Lösung für weitere 5 Stunden bei 30 °C gerührt. Anschliessend wird die Lösung auf 500 ml Eiswasser gegossen, wobei das Produkt ausfällt. Der ausgefallene Niederschlag wird abgesaugt, mit Wasser gewaschen und aus Isopropanol kristallisiert. Weisses Kristallisat mit Smp.: 131-132 °C

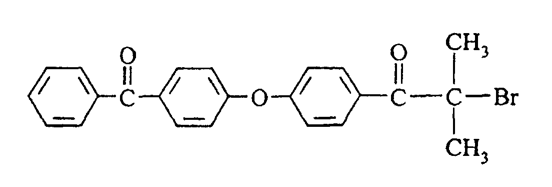

| | | | | | |
|---|---|---|---|---|---|
| Elementaranalyse | Ber. | C: 65.2 % | H: 4.5% | 0:11.3% | Br: 18.8% |
| | Gef. | C: 65.7 % | H: 4.5% | 0:11.7% | Br: 18.2% |

c) 8.9 g des Produktes aus der Stufe b) werden in 30 ml Isopropanol gelöst und auf 60 °C erwärmt, wobei eine klare Lösung entsteht. Dann wird die Lösung bei gleicher Temperatur während 3 Stunden mit einer Lösung aus 0.88 g NaOH in 10 ml Wasser tropfenweise versetzt, wobei das Reaktionsprodukt ausfällt. Der ausgefallene Niederschlag wird bei 5 °C abgesaugt und getrocknet. Weisses Kristallisat mit Smp. 113-114 °C

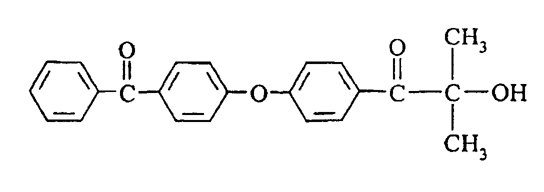

| | | | | |
|---|---|---|---|---|
| Elementaranalyse | Ber. | C: 76.6 % | H: 5.5 % | O: 17.7 % |
| | Gef. | C: 76.9 % | H: 5.4 % | 0: 17.7 % |

Analog zur Methode von Beispiel 1 werden auch die folgenden Beispiele der Tabelle hergestellt.

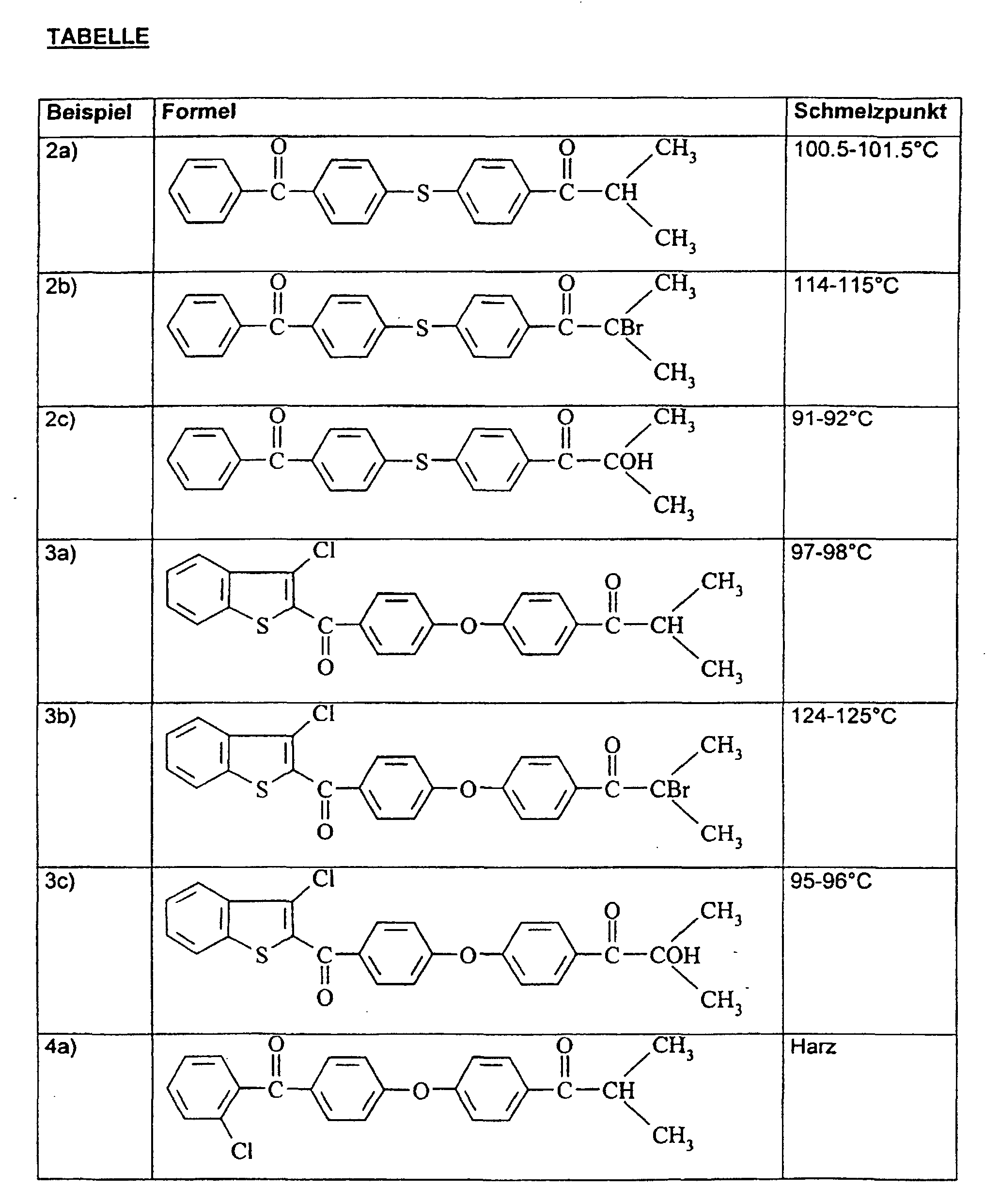

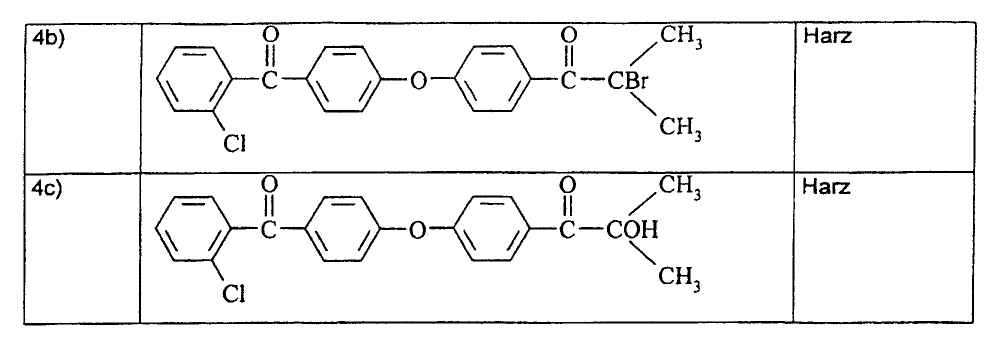

### ANWENDUNGSBEISPIEL 1

Eine Mischung bestehend aus 60 g eines Präpolymeren der Formel: 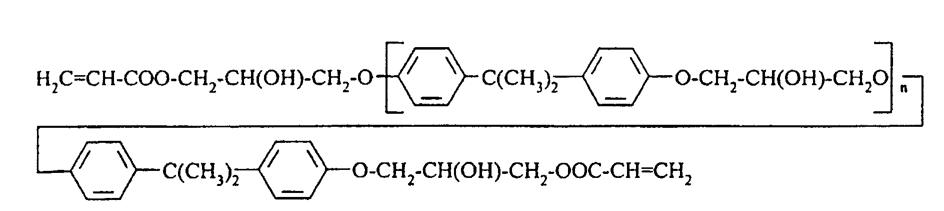 mit einer Viskosität von ca. 9000 poise (bei 77 °C), 35 g Pentaeritritoltetraacrylat und 5 g der Verbindung 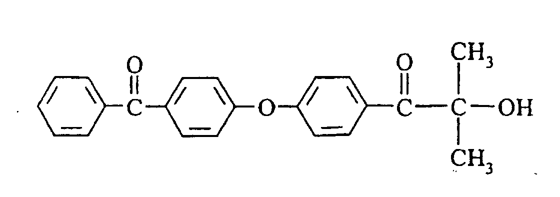 werden mit einem Spatel in einer Menge von 3.5 g/m² auf gestrichenem Kunstdruckpapier aufgebracht und im UV-Mini-Cure-Gerät bei einer Bandgeschwindigkeit von 72 m/Minuten zu einem glänzenden Film ausgehärtet.

### ANWENDUNGSBEISPIEL 2

Ein Fotoinitiator wird zu 3 Teilen mit 100 Teilen eines aminmodifizierten Polyätheracrylat (Viskosität bei 23° C: 600 mPas) - Laromer© PO 84F (BASF AG) - abgemischt.

Geprüft werden die Härtungsgeschwindigkeit als Bandgeschwindigkeit mit der ein Flüssiglackfilm, aufgetragen auf weissem Papier, unter einer undotierten Quecksilber-Hochdrucklampe (Leistung: 120 W/cm Lampenlänge; Lampenabstand zum Substrat 12 cm) durchgefahren werden kann, um eine gegenüber dem Fingernagel kratzfeste und haftende Beschichtung zu erhalten. Die Flüssiglackfilme werden mit einem 100 µm-Spiralrakel aufgetragen.

### ANWENDUNGSBEISPIEL 3

Ein Fotoinitiator wird zu 3 Teilen mit dem Gemisch aus 90 Teilen eines aminmodifizierten Polyacrylat (Viskosität bei 23° C: 600 mPas) - Laromer© PO 84F (BASF AG) - und 10 Teilen Anatas abgemischt. Bestimmt wird die durchhärtbare Schichtdicke. Dazu wird der in ein ca. 1 cm tiefes Gefäss gefüllte Lack mit 5 m/min Bandgeschwindigkeit bestrahlt. Die gehärtete Schicht wird mit Ethylacetat abgewaschen und deren Dicke bestimmt.

## Patentansprüche

1. Verbindungen der allgemeinen Formel (I) 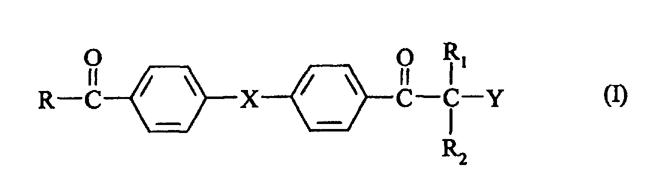 worin
R für Phenyl, mit C₁₋₄Alkyl, C₁₋₄Alkoxy oder Halogen substituiertes Phenyl, Naphthyl oder einen aromatischen Ring mit Heteroatomen steht;
X O, S bedeutet;
R₁ und R₂ je für einen Methylrest oder R₁ und R₂ gemeinsam für einen C₄₋₈Alkylenrest stehen;
Y Hydroxy, C₁₋₁₂Alkoxy, C₁₋₄Alkylamino, Di-C₁₋₄alkylamino oder ein am Stickstoff gebundenen Piperidin- oder Morpholinring bedeutet.

2. Verbindungen gemäss Patentanspruch 1, worin R für einen unsubstituierten oder mit Chlor oder C₁₋₄Alkyl substituierten Phenylring, X für Sauerstoff, R₁ und R₂ je für einen Methylrest und Y für Hydroxy oder Morpholin stehen.

3. Verfahren zur Herstellung von Verbindungen der allgemeinen Formel (I) gemäss Patentanspruch 1, **dadurch gekennzeichnet, dass** man Verbindungen der Formel (II) 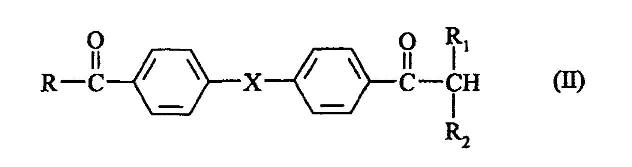 worin R, X, R₁ und R₂ die im Patentanspruch 1 für Formel (I) angegebenen Bedeutungen haben, halogeniert und den Halogenrest mit entsprechenden Verbindungen gegen die Gruppe Y austauscht.

4. Verbindungen der Formel (II) gemäss Patentanspruch 3.

5. Verfahren zur Herstellung von Verbindungen der Formel (II) gemäss Patentanspruch 3, **dadurch gekennzeichnet, dass** man Verbindungen der Formel (III) 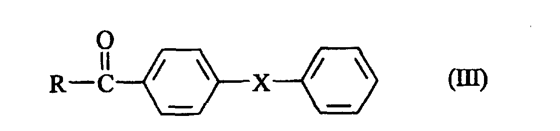 worin R und X die im Patentanspruch 1 für Formel (I) angegebenen Bedeutungen haben, mit Verbindungen der Formel (IV) 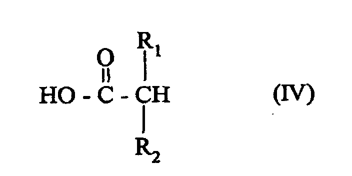 bzw. ihren reaktiven Derivaten, worin R₁ und R₂ die im Patentanspruch 1 angegebenen Bedeutungen haben, in Anwesenheit von passenden Katalysatoren, umsetzt.

6. Verwendung von Verbindungen der Formel (I) gemäss Patentanspruch 1 als Photoinitiatoren bzw. Photosensibilisatoren für photopolymerisierbare ungesättigte Verbindungen.

## Claims

1. Compounds of the general formula (I) 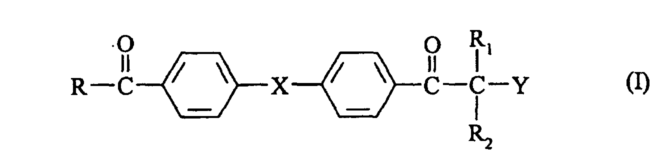 in which
R is phenyl, C₁₋₄alkyl-, C₁₋₄alkoxy- or halogen-substituted phenyl, naphthyl or an aromatic ring containing heteroatoms;
X is O, S;
R₁ and R₂ are each a methyl radical or R₁and R₂ together are a C₄₋₈alkylene radical;
Y is hydroxyl, C₁₋₁₂alkoxy, C₁₋₄alkylamino, di-C₁₋₄alkylamino or a piperidine or morpholine ring that is attached to nitrogen.

2. Compounds according to Claim 1 in which R is an unsubstituted or chloro- or C₁₋₄alkyl-substituted phenyl ring, X is oxygen, R₁ and R₂ are each a methyl radical and Y is hydroxyl or morpholine.

3. Process for preparing compounds of the general formula (I) according to Claim 1, **characterized in that** compounds of the formula (II) 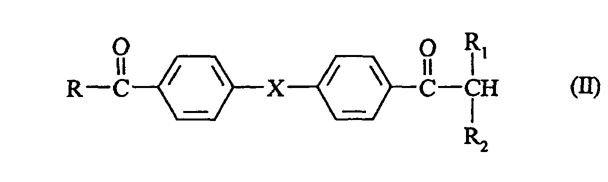 in which R, X, R₁ and R₂ are as defined in Claim 1 for formula (I) are halogenated and the halogen radical is exchanged for the group Y using corresponding compounds.

4. Compounds of the formula (II) according to Claim 3.

5. Process for preparing compounds of the formula (II) according to Claim 3, **characterized in that** compounds of the formula (III) 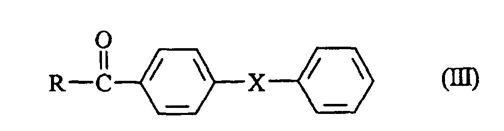 in which R and X are as defined in Claim 1 for formula (I) are reacted with compounds of the formula (IV) 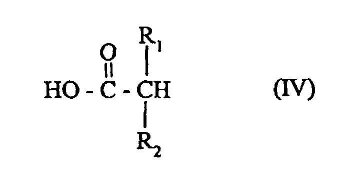 and/or their reactive derivatives, in which R₁ and R₂ are as defined in Claim 1, in the presence of appropriate catalysts.

6. Use of compounds of the formula (I) according to Claim 1 as photoinitiators and/or photosensitizers for photopolymerizable unsaturated compounds.

## Revendications

1. Composés de formule générale (I) 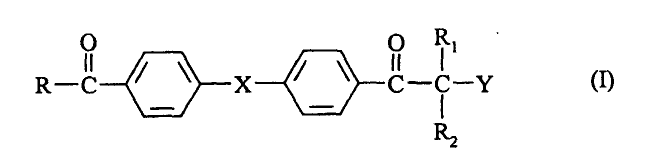 dans laquelle
R représente un phényle, un phényle substitué par alkyle en C₁ à C₄, alcoxy en C₁ à C₄ ou halogène, un naphtyle ou un cycle aromatique avec des hétéroatomes,
X signifie O, S,
R₁ et R₂ représentent chacun un radical méthyle ou R₁ et R₂ représentent ensemble un radical alkylène en C₄ à C₈,
Y signifie un hydroxy, un alcoxy en C₁ à C₁₂, un (alkyle en C₁ à C₄)-amino, un (dialkyle en C₁ à C₄)amino ou un cycle pipéridine ou morpholine lié par l'azote.

2. Composés selon la revendication 1, dans lesquels R représente un cycle phényle non substitué ou un cycle phényle substitué par chlore ou alkyle en C₁ à C₄, X représente un oxygène, R₁ et R₂ représentent chacun un radical méthyle et Y représente un hydroxy ou une morpholine.

3. Procédé pour la préparation de composés de formule générale (I) selon la revendication 1, **caractérisé en ce qu'**on halogène des composés de formule (II) 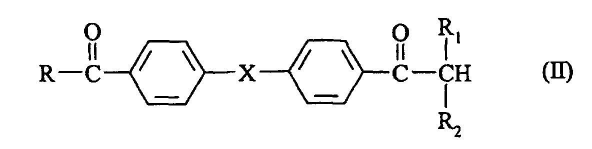 dans laquelle R, X, R₁ et R₂ ont les significations indiquées dans la revendication 1 pour la formule (I), et on remplace le radical halogène avec les composés correspondants par le groupement Y.

4. Composés de formule (II) selon la revendication 3.

5. Procédé pour la préparation de composés de formule (II) selon la revendication 3, **caractérisé en ce qu'**on transforme des composés de formule (III) 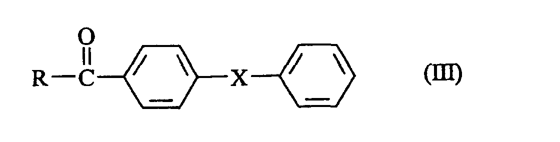 dans laquelle R et X ont les significations indiquées dans la revendication 1 pour la farmule (I), avec des composés de formule (IV) 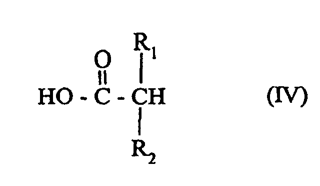 ou, selon le cas, leurs dérivés réactifs, dans laquelle R₁ et R₂ ont les significations indiquées dans la revendication 1, en présence de catalyseurs adaptés.

6. Utilisation des composés de formule (I) selon la revendication 1 comme photo-initiateurs ou, selon le cas, photo-sensibilisateurs pour des composés insaturés photopolymérisables.
